# EUROPEAN PATENT SPECIFICATION

(11) **EP 4 251 034 B1**
(45) Date of publication and mention of the grant of the patent: **07.01.2026**
(21) Application number: 21806296.6
(22) Date of filing: 22.11.2021
(51) Int. Cl.: A61B 5/022

(54) **SETTING DEVICE AND METHOD FOR A BLOOD PRESSURE MONITORING SYSTEM**
EINSTELLUNGSGERÄT UND VERFAHREN FÜR EIN BLUTDRUCKÜBERWACHUNGSSYSTEM
DISPOSITIF DE RÉGLAGE ET METHODE POUR UN SYSTÈME DE SURVEILLANCE DE LA PRESSION ARTÉRIELLE

(30) Priority: 30.11.2020 EP 20210694
(43) Date of publication of application: 04.10.2023
(73) Proprietor: Koninklijke Philips N.V., 5656 AG Eindhoven (NL)
(72) Inventor: SCHULZE, Andreas, 5656 AG Eindhoven (NL); WOEHRLE, Dieter, 5656 AG Eindhoven (NL); WUNDERLE, Norbert, Ernst, 5656 AG Eindhoven (NL); FALK, Jonas, 5656 AG Eindhoven (NL); FRANCK, Christoph, Florian, 5656 AG Eindhoven (NL)
(74) Representative: Philips Intellectual Property & Standards
(86) International application number: PCT/EP2021/082461
(87) International publication number: WO 2022/112156

(56) References cited:
- EP-A1- 0 913 121
- US-A1- 2007 129 636
- US-A1- 2014 039 824
- US-A1- 2014 073 951
- US-A1- 2017 290 520
- US-A1- 2018 078 158

## Description

### FIELD OF THE INVENTION

The present invention relates to a setting device and a method for a physiological parameter monitoring device configured to measure a physiological parameter of a patient, for instance for a blood pressure monitoring device configured to measure blood pressure of a patient. The present invention relates further to a physiological parameter monitoring system and a computer program.

### BACKGROUND OF THE INVENTION

Non-invasive blood pressure monitors using the oscillometric measurement method measure the systolic and diastolic blood pressure of a patient by inflating a cuff wrapped around one of the patient's limbs and measuring the amplitude of the pressure oscillations in the cuff caused by the changes in intra-arterial pressure that occur with each heartbeat. Generally, the oscillation amplitude is small when the cuff pressure is lower than the patient's diastolic pressure or higher than the patient's systolic pressure, and the oscillations are large if the cuff pressure is in the range between the patient's diastolic blood pressure and systolic blood pressure.

The oscillometric measurement is susceptible to artefacts that affect the pressure in the cuff. Such artefacts can be of physiological origin, for example by tremor or shivering of the patient, or of non-physiological origin, for example vibrations if the measurement is taken in a moving vehicle.

Other physiological parameter measurements than measurement of blood pressure may suffer from similar problems, in particular where the useful information is mostly carried in a measured signal by a fundamental frequency and its amplitude.

US 2014/073951 A1 discloses a physiological monitoring system that may process a physiological signal such a photoplethysmograph signal from a subject, determine physiological information, such as a physiological rate, from the physiological signal, use search techniques and qualification techniques to determine one or more initialization parameters, calculate and qualify a physiological rate, uses signal conditioning to reduce noise in the physiological signal and to improve the determination of physiological information, use qualification techniques to confirm determined physiological parameters, and use autocorrelation techniques, cross-correlation techniques, fast start techniques, and/or reference waveforms when processing the physiological signal.

US 2014/039824 A1 discloses a patient monitoring device and method that determines and monitors at least one patient parameter. A configuration processor generates configuration information in response to a first input signal and an adaptive notch filter receives a second input signal. The second input signal includes a signal of interest and an interference signal in a predetermined frequency range. The adaptive notch filter automatically estimates the interference signal within the second input signal based on a filter parameter and removes the estimated interference signal from the second input signal to generate a target signal. A step processor is electrically coupled between the configuration processor and the adaptive notch filter and sets a value of the filter parameter based on the configuration information, wherein the adaptive notch filter uses the filter parameter to reduce a ringing artifact on the target signal below a threshold level.

US 2007/129636 A1 discloses a vital sign monitoring system that can be used with multiple patients and utilizes historic patient data information for the patient to optimize the process of obtaining current vital sign measurements. Each patient is identified with a unique patient identification device that is automatically detected by the vital sign monitor. The vital sign monitor communicates with a medical records database and obtains historic patient data information for the patient identified by the patient identification device. The historic patient data information can be utilized by the vital sign monitor to set alarm limits for the vital sign measurements and automatically control the operation of an NIBP monitor for the specific patient.

### SUMMARY OF THE INVENTION

It is an object of the present invention to provide a setting device and method for a physiological parameter monitoring device configured to measure a physiological parameter of a patient to ensure that the measurements of the of the physiological parameter are less or even not affected by artefacts. It is a further object of the present invention to provide a corresponding physiological parameter monitoring system and a computer program.

The invention is defined by the independent claims. Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to configure a filter at the start of a measurement cycle, using the patient category (e.g. "adult" or "neonatal") and/or sensor or transducer information, e.g. the size of a cuff (as an example of a transducer) mounted to a patient's arm for non-invasive blood pressure measurement or the intended patient population (e.g. "adult" or "child") of a pulse oximetry sensor (as an example of a sensor) mounted to a patient's finger for SpO2 measurement. Patient category and such sensor or transducer information are indicators of the expected range of heart rates. Hereby, "sensor or transducer information" may be information about the sensor or information about the transducer applied in the respective embodiment.

The configuration of a filter can comprise selecting one of several filters (e.g. linear time-invariant (LTI) filters) or choosing a set of properties and constraints (e.g. for an adaptive filter). By appropriately configuring the filter, it can be ensured that measurement results of a physiological parameter monitor, e.g. a non-invasive blood pressure monitor or an SpO2 monitor, are affected as little as possible by artefacts.

The patient category may generally be obtained via user input. However, due to user error, the selected patient category may not reflect the actual patient so that the patient category may be obtained automatically, e.g. from a patient record. Another indicator of patient category is the size of the cuff used for the measurement. It is unlikely that a tiny cuff intended for neonates will be used on an adult patient, and it is likewise unlikely that a large cuff size intended for adult upper arms or thighs will be used on a neonate.

Generally, the present invention may be applied for all physiological parameters of a patient that can be measured by a sensor that provides a corresponding sensor signal. The signal to be filtered is generally a signal that carries the information mostly by a fundamental frequency and its amplitude. For instance, in case of photoplethysmographic sensors (including those with more than one wavelength like pulse oximetry), this will be the waveforms produced by the photosensor(s) of the photoplethysmographic sensors.

According to an embodiment the data input is configured to obtain the patient category information via user input or from a database holding patient data. The data input may e.g. be provided via a keyboard, touchpad or speech recognition interface. Alternatively, a central database of patient records may be accessed to obtain (i.e. receive or retrieve) the patient category information from the record of the current patient. The patient category information may include one or more of the age of the patient, the age category (e.g. "neonate", "child", "adult", "senior", etc.), and the gender of the patient.

According to another embodiment the data input is configured to obtain the sensor or transducer information via user input or by detecting the type and/or size of the sensor or transducer. The user may thus actively input such information via a user interface or the information may be detected, e.g. by reading a barcode or RFID tag storing sensor or transducer information arranged on the sensor or transducer or by otherwise obtaining such information from the sensor or transducer that may e.g. store such information on an accessible memory or storage element.

According to another embodiment the data input is configured to additionally obtain the expected range of the patient's heart rate via user input and the heart rate estimation unit is configured to determine the expected range of the patient's heart rate from a comparison of the estimated range and the expected range obtained via user input. A user may thus manually input an expected range of the current patient's heart rate, e.g. based on his general experience or based on prior knowledge about this particular patient (e.g. from prior heart rate measurements). This input is then used to improve the estimated range and determine a more precise range used in the further processing.

The setting unit may be configured to select a digital filter, a linear time-invariant (LTI) filter or an adaptive filter. For example, an LTI filter may be used for "neonatal" patients, and an adaptive filter may be used for "adult" patients. This can be advantageous if the adaptive filter has only been clinically validated for adult patient populations and may not be used when measuring a physiological parameter of neonatal patients.

The setting unit may further be configured to generate as one or more settings one or more of corner frequency of a low-pass filter or high-pass filter, corner frequencies of a band-pass filter, filter order, filter type, maximum passband ripple, stopband ripple, window function, adaptation algorithm, upper and/or lower bounds on filter coefficient values, upper bounds for l2 filter norm, one or more constraints that force certain gains at certain frequencies, and function to be minimized and/or maximized by the filter.

For instance, in an embodiment the setting unit may be configured, in case the patient category is adult, to generate as setting an upper corner frequency of the filter in a range between 4 Hz and 5 Hz in case the sensor or transducer size exceeds a size threshold and to generate as setting an upper corner frequency of the filter in a range above 5 Hz in case the sensor or transducer size is below a size threshold, in particular to generate, in case the patient category is neonatal or toddler, as setting an upper corner frequency of the filter in a range above 5 Hz. This improves the accuracy of the measured physiological parameter and supports removal of artefacts.

**In** another embodiment the setting unit may be configured to select a filter from a group of predefined filters or to select a set of settings from a group of sets of settings based on the expected range of the patient's heart rate. There may e.g. be a list or table of predefined filter and/or sets of setting for different ranges of heart rates (and optionally one or more further parameters, such as patient category information and sensor or transducer information). This enables an easily implementable solution.

According to a further embodiment the setting unit is configured to use the patient category information and/or the sensor or transducer information to generate one or more settings of the filter, i.e., in addition to the expected range of the patient's heart rate the patient category information and/or the sensor or transducer information may be used to generate one or more settings of the filter. It may even be possible to directly use, instead of the expected range of the patient's heart rate, the patient category information and/or the sensor or transducer information to generate one or more settings of the filter, in which case the estimation of the expected range of the patient's heart rate from the obtained patient category information and/or the obtained sensor or transducer information may even be completely omitted.

In a particular application of the present invention for non-invasive blood pressure measurement, the physiological parameter monitoring device is a blood pressure monitoring device configured to measure blood pressure of a patient, the transducer is a cuff, the transducer information includes cuff size information indicating cuff size of a cuff, and the filter is configured to filter a cuff pressure signal measured by the blood pressure monitoring device. In such an application the data input may practically be configured to detect the size of the cuff by monitoring the change in cuff pressure in response to a particular volume of air pumped into the cuff or by identifying the size from an identifier arranged in or at the cuff.

The presented physiological parameter monitoring system generally comprises a sensor configured to acquire a sensor signal from the patient, a filter configured to filter the measured sensor signal, a processor configured to determine a physiological parameter of the patient based on the filtered sensor signal, and a setting device as described above for configuring the filter. In the application for blood pressure measurement mentioned above, the sensor is a pressure sensor configured to measure the cuff pressure and generate a cuff pressure signal, the filter is configured to filter the cuff pressure signal measured by the blood pressure monitoring device, the physiological parameter monitoring device further comprises a pressure generating unit configured to inflate the cuff that is configured to be attached to a patient's body part and a valve configured to deflate the cuff, and the processor is configured to control the pressure generating unit and the valve and to determine the patient's blood pressure based on the filtered cuff pressure signal.

One embodiment of the present invention for suppressing or rejecting artefacts is applying a digital filter to the digitally sampled cuff pressure, which will attenuate components of the pressure signal outside the relevant frequency band. The behavior of such a filter, especially its corner frequencies, can be chosen in an embodiment so that the filter does not attenuate signal components relevant for blood pressure calculation. This may be achieved by setting one or more the filter's properties on the expected heart rate range of the patient since different categories of patients, for example "neonates" or "adults", generally will have different heart rate ranges.

Generally, the sensor may be a physiological parameter sensor that is configured to be attached to a patient's body part, in particular a heart rate sensor, a respiration rate sensor or a pulse oximetry sensor, and/or the transducer may a non-invasive blood pressure cuff, an ultrasound transducer, a photoplethysmographic transducer or a direct pressure transducer. The filter may be a digital filter, a linear time-invariant filter or an adaptive filter.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a physiological parameter monitoring system according to the present invention;
Fig. 2 shows a schematic diagram of a second embodiment of a physiological parameter monitoring according to the present invention;
Fig. 3 shows a schematic diagram of a setting device according to the present invention;
Fig. 4 shows a schematic diagram of an embodiment of a blood pressure monitoring system including an embodiment of a blood pressure monitoring device according to the present invention;
Fig. 5 shows a flow chart of an embodiment of a physiological parameter monitoring according to the present invention;
Fig. 6 shows a flow chart of an embodiment of a blood pressure monitoring according to the present invention;
Fig. 7 shows a flow chart of another embodiment of a blood pressure monitoring method 60 according to the present invention; and
Fig. 8 shows diagrams illustrating the effect of non-invasive blood pressure monitoring according to the present invention.

### DETAILED DESCRIPTION OF EMBODIMENTS

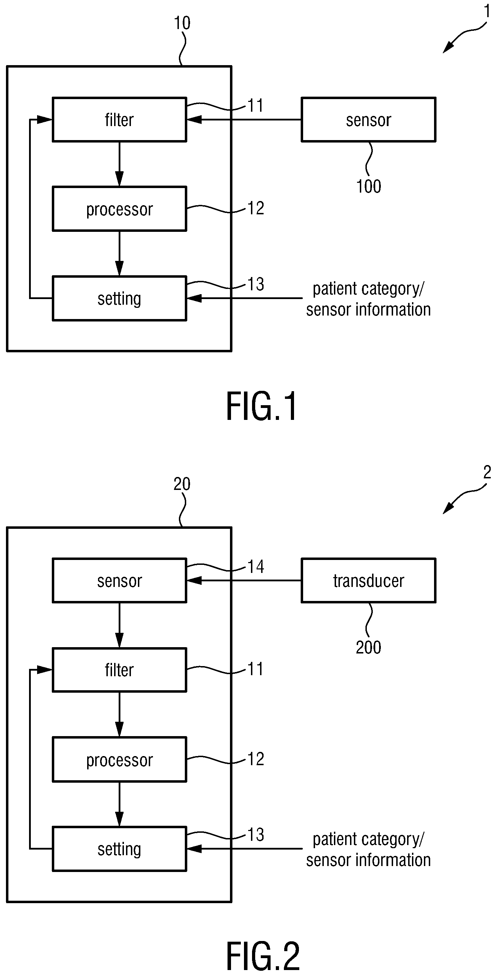

Fig. 1 shows a schematic diagram of a first embodiment of a physiological parameter monitoring system 1 according to the present invention for measuring a physiological parameter of a patient. The system 1 includes a sensor 100 for acquiring a sensor signal from the patient, a filter 11 for filtering the measured sensor signal, a processor 12 for determining a physiological parameter of the patient based on the filtered sensor signal, and a setting device 13 for configuring the filter 11.

In this embodiment the filter 11, the processor 12 and the setting device 13 are part of a physiological parameter monitor 10, such as a patient monitor. The sensor 100 is a physiological parameter sensor external to the monitor 10 and is configured to be attached to a patient's body part (e.g. the patient's finger, arm, chest, neck, etc.). The sensor 100 may e.g. be a heart rate sensor for measuring heart rate for measuring heart rate (e.g. from light reflections of the skin using photoplethysmography), a respiration rate sensor for measuring respiration rate (e.g. from breathing movements of the chest or neck) or a pulse oximetry sensor for measuring SpO2 (e.g. from reflected light in the red and infrared spectrum in response to light emitted onto e.g. the finger).

In this context it shall be noted that the term "heart rate" shall be understood either as the conventional heart rate (i.e., the number of times per minute that the heart contracts or the number of heart beats per minute) or the pulse rate (i.e., the rate of the mechanical pulse of blood flow through the capillaries caused by the contractions of the heart per minute).

Fig. 2 shows a schematic diagram of a second embodiment of a physiological parameter monitoring system 2 according to the present invention. In this embodiment the system 2 generally comprises the same elements as the system 1, but the sensor 14 is part of the physiological parameter monitor 20 that also comprises the filter 11, the processor 12 and the setting device 13.

Further, the system 2 comprises a transducer 200 that is external to the monitor 20 and is configured to be attached to a patient's body part (e.g., the patient's finger, arm, chest, neck, etc.). The transducer 200 may e.g. be an NiBP cuff, an ultrasound transducer for measuring heart movements or flow velocity in blood vessels, a photoplethysmographic transducer using one or several wavelengths (e.g. two wavelengths as required for pulse oximetry, but optionally more wavelengths for acquiring additional information), or a direct pressure transducer (e.g., for invasive measurements of intra-arterial or intravenous pressure).

In such a system 2 the sensor 14 may be a pressure sensor for measuring the pressure and generating a pressure signal, an ultrasound sensor for receiving measured ultrasound or flow velocity information and generating an ultrasound or flow velocity signal, or a photoplethysmography sensor for receiving measured optical information and generating a heart rate or SpO2 signal.

The filter 11, the processor 12 and the setting device 13 may generally be implemented by a common processing element (e.g. a processor or computer) that is programmed accordingly to carry out the functions of the respective units. In other embodiments, separate processing elements or other hard- and/or software may be provided to implement these units.

The physiological parameter monitors 10, 20 may comprise, although not explicitly shown, further components such as a user interface for input and/or output of information (e.g. a keyboard, touchpad, screen or display), a controller for controlling operations like data acquisition, data processing, data output, signal processing means like an analog-to-digital converter, etc.

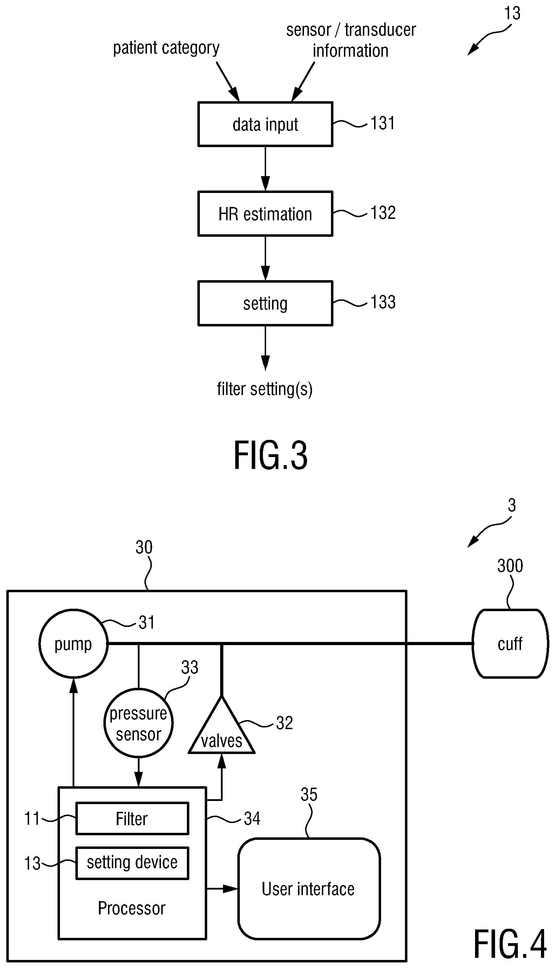

For setting the filter the setting device 13 uses patient category information and/or sensor or transducer information as input as shown in Figs. 1 and 2. This will be explained below with reference to Fig. 3 showing a schematic diagram of a setting device 13 according to the present invention. It comprises a data input 131, a heart rate estimation unit 132 and a setting unit 133.

The data input 131, e.g. user interface or a (wireless or wired) data interface, obtains patient category information indicating the patient category of the patient and/or sensor or transducer information indicating one or more of type, size, shape, attachment location or intended patient population of the sensor 100 or the transducer 200 used in the physiological parameter monitoring.

The heart rate estimation unit 132, e.g. a processing or computing element, estimates an expected range of the patient's heart rate from the obtained patient category information and/or the obtained sensor or transducer information.

The setting unit 133, e.g. a processing or computing element, generates one or more settings of the filter 11 based on the expected range of the patient's heart rate (and, optionally in addition the patient category information and/or the sensor or transducer information). Further, the setting unit 133 configures the filter 11 for filtering a sensor signal measured by the physiological parameter monitoring device 20 (in particular the sensor 14) or the sensor 100 based on the generated settings.

Generally, the sensor or transducer information may include one or more of size, type, shape, attachment location (e.g. ear, finger, foot, etc.) or intended patient population (e.g., if the sensor or transducer includes a digital memory for storing and retrieving such information). For instance, there are special neonatal SpO2 sensors that are not used on adult patients, i.e. such a special neonatal SpO2 sensor may confer sensor information or allow reading such information from the sensor.

Fig. 4 shows a schematic diagram of a more detailed embodiment for the application in blood pressure monitoring, i.e., it shows a blood pressure monitoring system 3 including an embodiment of a blood pressure monitoring device 30 according to the present invention. The non-invasive blood pressure monitoring device 30 particularly uses the oscillometric measurement method and controls an NiBP cuff 300 (representing the external transducer 200 of the system 2 shown in Fig. 2) that is arranged at the upper part of the patient's arm and thus non-invasively measures the patient's blood pressure.

For this purpose, the NiBP cuff 300 is connected to the blood pressure monitoring device 30 by a pneumatic connection. Optionally, a digital connection may additionally be provided to read transducer information from the NiBP cuff 300 for use in configuration of the filter 11. The NiBP cuff 300 may thus optionally comprise a digital memory in this embodiment.

The blood pressure monitoring device 30 comprises a pressure generating unit 31, e.g. a pump, that is configured to inflate the cuff 300, a valve 32 that is configured to deflate the cuff 300, a pressure sensor 33 (representing the sensor 14 of the monitor 20 shown in Fig. 2) that is configured to measure the cuff pressure, and a processor 34 (representing the processor 12 of the monitor 20 shown in Fig. 2) configured to control the pressure generating unit 31 and the valve 32 and to determine the patient's blood pressure based on the measured cuff pressure. Further, the filter 11 for filtering the cuff pressure signal measured by the pressure sensor 33 and the setting device 13 for configuring the filter 11 are provided. A user interface 35 may be provided, e.g. comprising one or more of a display, keypad, loudspeaker and touchpad, to issue the measured blood pressure. Optionally, a (digital) interface (not shown) to the NiBP cuff 300 may be provided to obtain transducer information from the optional memory of the NiBP cuff 300.

The filter 11 and the setting device 13 may, as shown in Fig. 3, be part of the processor 34 that is programmed accordingly to carry out the functions of these components. In other embodiments, a separate processor or other hard- and/or software may be provided to implement these components. The filter 11 and the setting device 13 may thus also be provided as separate units external from the processor 34 or even external from the blood pressure monitoring device 30.

The processor 34 may thus perform the following operations: control pump and valves, acquire signal(s) from pressure sensor, configure filter based on patient category and/or cuff size, digital signal processing (apply selected filter to pressure signal), and calculate NiBP values from filtered pressure signal.

The user interface 35 may perform the following operations: set patient category, start measurement or set automatic measurement, and display values and notifications.

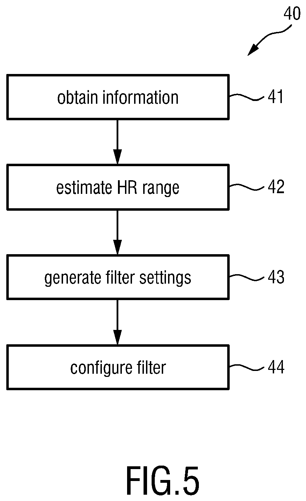

Fig. 5 shows a flow chart of an embodiment of a physiological parameter monitoring method 40 according to the present invention that may be carried out by a physiological parameter monitoring system 1 or 2 shown in Figs. 1 and 2. In a first step 41 patient category information indicating the patient category of the patient and/or sensor or transducer information of the sensor 100 or transducer 200, 300 is obtained. In a second step 42 an expected range of the patient's heart rate is estimated from the obtained patient category information and/or the obtained sensor or transducer information. In a third step 43 one or more settings of the filter 11 are determined based on the expected range of the patient's heart rate and/or the patient category information and/or the sensor or transducer information. In a fourth step 44 the filter 11 is configured for filtering a sensor signal measured by the physiological parameter monitoring device 10, 20, 30 or the sensor 100, 14, 33 based on the generated settings.

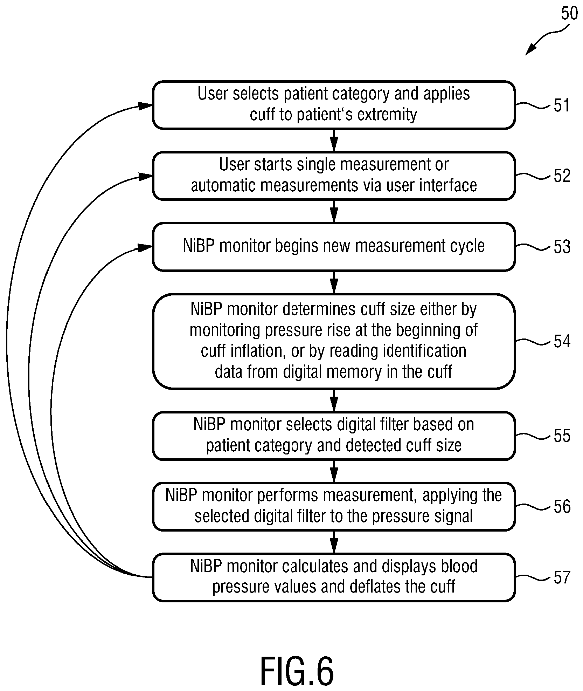

Fig. 6 shows a flow chart of an embodiment of a blood pressure monitoring method 50 according to the present invention that may be carried out by use of the blood pressure monitoring system 3 shown in Fig. 4. In a first step 51 a user (e.g. a nurse or doctor) selects the patient category and applies the cuff to a patient's extremity. In a second step 52 the user starts a single measurement or automatic measurements via the user interface 35. In a third step 53 the NiBP monitor 30 begins a new measurement cycle. In a fourth step 54 the NiBP monitor 30 determines the cuff size, e.g. by monitoring pressure rise at the beginning of cuff inflation or by reading identification data from a digital memory in the cuff. In a fifth step 55 the NiBP monitor 30 selects a digital filter (which is one option of configured the filter 11) based on patient category and detected cuff size. In a sixth step 56 the NiBP monitor 30 performs a measurement, applying the selected digital filter to the pressure signal. In a seventh step 57 the NiBP monitor 30 calculates and displays blood pressure values and deflates the cuff.

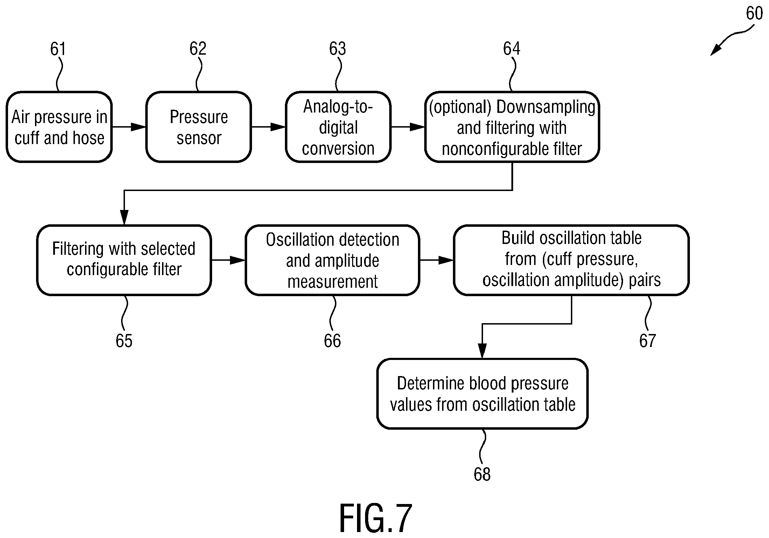

Fig. 7 shows a flow chart of another embodiment of a blood pressure monitoring method 60 according to the present invention that may be carried out by use of the blood pressure monitoring system 3 shown in Fig. 4. This flow chart particularly illustrates the signal processing operations. In a first step 61 the cuff is inflated to increase the pressure in the cuff and the hose. In a second 62 the pressure is measured while deflating the cuff. In a third step 63 the measured pressure signal is subjected to analog-to-digital conversion. In a fourth step 64 an option down sampling and filtering (using a non-configurable) filter are performed. In a fifth step 65 filtering with the selected / configured filter is performed. In a sixth step 66 oscillation detection and amplitude measurement are performed. In a seventh step 67 an oscillation table is formed from pairs of cuff pressure and oscillation amplitude. In an eighth step 68 the blood pressure values are determined from the oscillation table.

As explained above, the monitor 30 may allow the user to select a patient category from a set (e.g. "neonatal", "pediatric", "adult"). Further, the monitor 30 may support different cuff sizes and can detect the size of the connected cuff. The detection may be performed by monitoring the change in cuff pressure in response to a certain volume of air pumped into the cuff at the beginning of cuff inflation or by having a means of directly identifying the size, such as a digital memory device built into the cuff that can be read by the monitor by a wired or wireless (e.g. RFID) connection. At the beginning of a measurement cycle, the monitor 30 uses the selected patient category and/or the detected cuff size to configure the properties of the (preferably digital) filter that will be applied to the measured cuff pressure signal when the monitor is detecting oscillations during the measurement.

The present invention provides for an improved robustness of the physiological parameter measurement, e.g. of a non-invasive blood pressure measurement, against artefacts, resulting in more accurate physiological parameter values even when artefacts are present. By choosing/configuring a filter based on the expected heart rate range, the physiological parameter monitor can reject undesired artefact components above this range (e.g. physiological temperature-related shivering in a frequency range from 5 Hz to 8 Hz) and below this range (e.g., in the application of blood pressure measurement: pressure fluctuations caused by respiration if the cuff is in contact with the patient's torso).

By coupling the filter selection to the user-selectable patient category and/or the sensor or transducer information (e.g. the cuff size for blood pressure measurements), it can happen automatically at the start of the measurement without requiring the user, who is probably not versed in signal processing, to manually configure a filter.

In a simple embodiment, the configuration may comprise choosing one linear time-invariant (LTI) filter from a set of predefined filters depending on patient category and/or sensor or transducer information. For instance, if the patient category is "adult" (i.e., using patient population information), a filter with 4.5 Hz upper corner frequency may be chosen. If the patient category is "neonatal", a filter with 5.5 Hz upper corner frequency may be chosen. The reason is that the expected heart rate range for an adult is 30 to 210...240 bpm (beats per minute) corresponding to a frequency of 0.5 to 3.5...4 Hz). The upper corner frequency of filter may thus be n times 4...4.5 Hz (n=1, 2, 3, ... depending on how much of the high-frequency content is relevant for the measurement). For neonates the expected heart rate range is 30 to 300 bpm (0.5 ... 5 Hz). The upper corner frequency of the filter may thus be n times 5...5.5 Hz.

In an advanced embodiment, the physiological parameter monitor may use adaptive filters. Adaptive filters change their behavior in response to the input data and do not have explicitly configurable corner frequencies. However, they still have properties like filter order, adaptation algorithm (e.g. LMS, RLS, block-wise), and constraints that are applied to their coefficients or output values. Such constraints may be equalities or inequalities of the amplitude gain of the filter at certain frequencies, or constrain a norm of the filter coefficients to be equal to or not greater than a given value. This way, certain properties of the adaptive filter can be ensured even as the adaptation algorithm changes the coefficients of the filter. In this advanced embodiment, the physiological parameter may choose from a set of filter properties and constraints based on the selected patient category and/or sensor or transducer information.

In an embodiment the monitor may also choose between LTI filters and adaptive filters depending on patient category and sensor or transducer information. For example, an LTI filter may be used for "neonatal" patients, and an adaptive filter may be used for "adult" patients. This can be advantageous if the adaptive filter has only been clinically validated for adult patient populations and may not be used when measuring physiological parameters of neonatal patients.

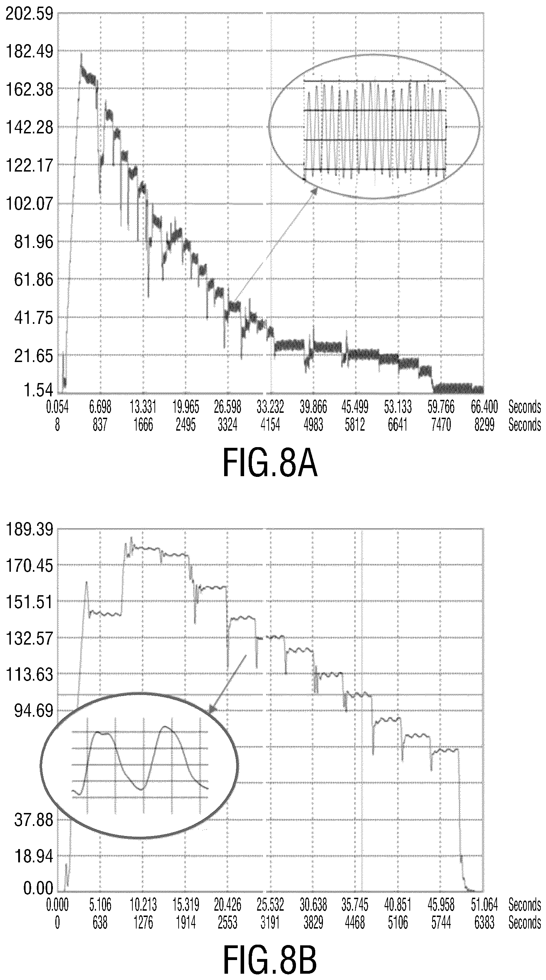

Fig. 8 shows diagrams illustrating the effect of non-invasive blood pressure monitoring according to the present invention.

Fig. 8A shows a diagram of the measured blood pressure signal over time using a fixed filter with comparatively high corner frequency. As shown in the enlarged visualization of the blood pressure signal in this diagram, the physiological signal is almost invisible, and the stepwise deflation of the cuff continues to values far below the diastolic blood pressure.

Fig. 8B shows a diagram of the measured blood pressure signal over time using a configurable filter that is configured according to the present invention so that it is more suited for the patient category and has a lower corner frequency. As shown in the enlarged visualization of the blood pressure signal in this diagram, the physiological signal is clearly visible.

In the following some further exemplary embodiments will be explained that illustrate the relationship between one or more settings of the filter and one or more pieces of sensor or transducer information.
i) The corner frequency of a high-pass filter may be set as follows:
   adult: 0.4167 Hz (25 bpm, to cover rare cases of extreme bradycardia in adults);
   neonatal: 0.5 Hz (30 bpm, as neonates have a higher heart range on average).
ii) The corner frequencies of band-pass filter may be set as follows:
   adult: 0.4167 Hz - 3.3333 Hz (25 bpm - 210 bpm, to cover the expected heart rate of adults including some pathological cases);
   neonatal: 0.5 Hz - 4.167 Hz (30 bpm - 250 bpm).
iii) The filter order may be set as follows:
   adult: 3rd or 4th order (for faster transition into stop band and better rejection of frequencies outside the signal band);
   neonatal: 1st or 2nd order (slower transition into stop band, as for example shivering is less frequently an issue).
iv) The filter type may be set as follows:
   adult: Adaptive filter (that was validated using adult patients, suppresses artefacts like shivering that are less of an issue with neonatal patients);
   neonatal: Linear time-invariant filter (if the adaptive filter was only validated with an adult patient population).
v) The maximum passband ripple may be set as follows:
   adult: 0.25 dB (heart rate is not expected to vary rapidly; allowing some passband ripple can improve other desirable properties like the steepness of transition into the stopband);
   neonatal: 0.1 dB (heart rate is expected to have more variability, and signal is generally smaller than in an adult patient).
vi) The stopband ripple may be set as follows:
   adult: Stopband has at least -50 dB attenuation (strong suppression of artefacts like shivering or vibrations from moving vehicles);
   neonatal: Stopband has at least -40 dB attenuation (trading noise suppression for other desirable properties like filter settling time).
vii) A window function (for symmetric finite impulse response filter designs) may be set as follows:
   adult: Hamming window or rectangular window (faster transition into the stopband, but more ripple in the stopband);
   neonatal: Blackman or Hanning window (slower transition into the stopband, but less ripple in the stopband).
viii) An adaptation algorithm (if more than one patient category makes use of adaptive filters) may be set as follows:
   adult: "RLS", recursive least squares;
   neonatal: "LMS", least mean-squares (if the RLS algorithm was only validated with an adult patient population and the LMS algorithm was validated with a neonatal patient group).
ix) Upper and/or lower bounds on filter coefficient values (adaptive filter only) may be set as follows:
   adult: limit to the interval [-2.0, 2.0], this gives the optimization algorithm more degrees of freedom, for example by allowing some high-pass behavior of the filter;
   neonatal: limit to the interval [0, 2.0], this limits to optimization algorithm to low-pass behavior only.
x) Upper bound for l2 filter norm (adaptive filter only) may be set as follows:
   adult: l2norm <= 0.4;
   neonatal: l2norm <= 0.5 (this allows the resulting filter to have slightly lower mean attenuation than for the adult patient category; a lower mean attenuation allows for a larger bandwidth).
xi) Constraints that force gains at certain frequencies (adaptive filter only) may be set as follows:
   adult: Gain greater than -3dB at 4 Hz (240 bpm), gain less than -20 dB at 6 Hz (smaller pass-band, sharper transition into stop band);
   neonatal: Gain greater than -3 dB at 5 Hz (300 bpm), gain less than -20 dB at 8 Hz (wider pass-band, slower transition into the stop band).
xii) A function to be minimized and/or maximized by the filter (adaptive filter only) may be set as follows:
   adult: minimize the power of the signal in the noise frequency band (e.g. 5 Hz to 50 Hz at 100 Hz sampling rate) with more emphasis on the frequencies from 5 Hz to 11 Hz (physiological shivering);
   neonatal: minimize the power of the signal in the noise frequency band (e.g. 5 Hz to 50 Hz at 100 Hz sampling rate) while weighing all frequencies equally.
xiii) Filter settings for e.g. an SpO2 sensor may be made as follows:
   sensor type is a neonatal foot sensor: use filter settings for neonatal patient;
   sensor type is ear sensor: use filter settings for adult/pediatric patient.
xiv) Filter settings based on the attachment location may be made as follows:
   cuff attached to the arm of the patient: may experience physiological shivering at a higher frequency range; use filter settings to filter out effects from shivering at higher frequency range.
   cuff attached to the thigh of the patient: may pick up physiological shivering at a lower frequency range; use filter settings to filter out effects from shivering at lower frequency range.

Thus, the attachment location may also provide a hint to the parameters of the interference that is to be expected. Depending on the attachment location shivering in different frequency ranges may be expected, e.g. because of differences in mass and muscle structure, in this example between arm and leg.

In other embodiments size and/or shape of the sensor or transducer may be considered in the settings of the filter. For instance, the size may, similar to age or patient population information, indicate for which patient the sensor or transducer is configured (e.g. for an adult, teenager, young child or neonatal). The shape may also indicate the age or patient population or may indicate the attachment location so that the above-mentioned examples mutually apply for those pieces of sensor or transducer information.

It shall be noted that the above-mentioned embodiments and numbers are to be understood as examples only and that other number may be used in other embodiments.

The present invention may generally be applied for measuring one or more physiological parameter. In may particularly be applied in non-invasive blood pressure monitors, either as standalone devices or integrated into a larger system like a patient monitor.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Setting device (13) for configuring a filter (11) for a physiological parameter monitoring device (10, 20, 30) configured to measure a physiological parameter of a patient, the setting device comprising:
- a data input (131) configured to obtain patient category information indicating the patient category of the patient and/or sensor or transducer information indicating one or more of type, size, shape, attachment location or intended patient population of a sensor (100) or transducer (200, 300) used in the physiological parameter monitoring,
- a heart rate estimation unit (132) configured to estimate an expected range of the patient's heart rate from the obtained patient category information and/or the obtained sensor or transducer information, and
- a setting unit (133) configured to generate one or more settings of the filter (11) based on the expected range of the patient's heart rate and to configure the filter (11) for filtering a sensor signal measured by the physiological parameter monitoring device (10, 20, 30) or the sensor (100) based on the generated settings.

2. Setting device as claimed in claim 1,
wherein the data input (131) is configured to obtain the patient category information via user input or from a database holding patient data.

3. Setting device as claimed in any one of the preceding claims,
wherein the data input (131) is configured to obtain the sensor or transducer information via user input or by detecting the type and/or size of the sensor or transducer.

4. Setting device as claimed in any one of the preceding claims,
wherein the data input (131) is configured to additionally obtain the expected range of the patient's heart rate via user input and
wherein the heart rate estimation unit (132) is configured to determine the expected range of the patient's heart rate from a comparison of the estimated range and the expected range obtained via user input.

5. Setting device as claimed in any one of the preceding claims,
wherein the setting unit (133) is configured to select a digital filter, a linear time-invariant filter or an adaptive filter or to select a filter from a group of predefined filters or to select a set of settings from a group of sets of settings based on the expected range of the patient's heart rate.

6. Setting device as claimed in any one of the preceding claims,
wherein the setting unit (133) is configured to generate as one or more settings one or more of corner frequency of a low-pass filter or high-pass filter, corner frequencies of a band-pass filter, filter order, filter type, maximum passband ripple, stopband ripple, window function, adaptation algorithm, upper and/or lower bounds on filter coefficient values, upper bounds for l2 filter norm, one or more constraints that force certain gains at certain frequencies, and function to be minimized and/or maximized by the filter.

7. Setting device as claimed in any one of the preceding claims,
wherein the setting unit (133) is configured, in case the patient category is adult, to generate as setting an upper corner frequency of the filter in a range between 4 Hz and 5 Hz in case the sensor or transducer size exceeds a size threshold and to generate as setting an upper corner frequency of the filter in a range above 5 Hz in case the sensor or transducer size is below a size threshold, in particular to generate, in case the patient category is neonatal or toddler, as setting an upper corner frequency of the filter in a range above 5 Hz.

8. Setting device as claimed in any one of the preceding claims,
wherein the physiological parameter monitoring device is a blood pressure monitoring device (30) configured to measure blood pressure of a patient, the transducer is a cuff (300), the transducer information includes cuff size information indicating cuff size of a cuff (300), and the filter (11) is configured to filter a cuff pressure signal measured by the blood pressure monitoring device (30); and
wherein the data input (131) is configured to detect the size of the cuff by monitoring the change in cuff pressure in response to a particular volume of air pumped into the cuff or by identifying the size from an identifier arranged in or at the cuff.

9. Setting device as claimed in any one of the preceding claims,
wherein the setting unit (133) is configured to use the patient category information and/or the sensor or transducer information to generate one or more settings of the filter (11).

10. Computer-implemented method for configuring a filter (11) in a physiological parameter monitoring device (10, 20, 30) configured to measure a physiological parameter of a patient, the method comprising:
- obtaining patient category information indicating the patient category of the patient and/or sensor or transducer information indicating one or more of type, size, shape, attachment location or intended patient population of a sensor (100) or transducer (200, 300) used in the physiological parameter monitoring,
- estimating an expected range of the patient's heart rate from the obtained patient category information and/or the obtained sensor or transducer information,
- generating one or more settings of the filter (11) based on the expected range of the patient's heart rate, and
- configuring the filter (11) for filtering a sensor signal measured by the physiological parameter monitoring device (10, 20, 30) or the sensor (100) based on the generated settings.

11. Physiological parameter monitoring system (1, 2, 3) comprising:
- a sensor (100, 14, 33) configured to acquire a sensor signal from a patient;
- a filter (11) configured to filter the sensor signal;
- a physiological parameter monitoring device (10, 20, 30) comprising a processor (12, 34) configured to determine a physiological parameter of the patient based on the filtered sensor signal; and
- a setting device (13) as claimed in any one of claims 1 to 9 configured to configure the filter (11).

12. Physiological parameter monitoring system as claimed in claim 11, wherein
the physiological parameter monitoring device is a blood pressure monitoring device (30) configured to measure blood pressure of a patient,
the transducer is a cuff (300),
the transducer information includes cuff size information indicating cuff size of a cuff (10),
the sensor (33) is a pressure sensor configured to measure the cuff pressure and generate a cuff pressure signal,
the filter (11) is configured to filter a cuff pressure signal measured by the blood pressure monitoring device (30),
the physiological parameter monitoring device (30) further comprises a pressure generating unit (31) configured to inflate the cuff (300) that is configured to be attached to a patient's body part and a valve (32) configured to deflate the cuff, and
the processor (34) is configured to control the pressure generating unit (31) and the valve (32) and to determine the patient's blood pressure based on the filtered cuff pressure signal.

13. Physiological parameter monitoring system as claimed in claim 11,
wherein the sensor (100) is a physiological parameter sensor that is configured to be attached to a patient's body part, in particular a heart rate sensor, a respiration rate sensor or a pulse oximetry sensor.

14. Physiological parameter monitoring system as claimed in claim 11 or 13,
wherein the transducer (200, 300) is a non-invasive blood pressure cuff, an ultrasound transducer, a photoplethysmographic transducer or a direct pressure transducer.

15. Computer program comprising program code for causing a computer to carry out the method as claimed in claim 10 when said computer program is carried out on the computer.

## Patentansprüche

1. Einstellvorrichtung (13) zum Konfigurieren eines Filters (11) für eine Vorrichtung (10, 20, 30) zur Überwachung physiologischer Parameter, die zum Messen eines physiologischen Parameters eines Patienten konfiguriert ist, wobei die Einstellvorrichtung umfasst:
- eine Dateneingabe (131), die dazu konfiguriert ist, Patientenkategorieinformationen, die die Patientenkategorie des Patienten angeben, und/oder Sensor- oder Wandlerinformationen zu erhalten, die eines oder mehrere von Typ, Größe, Form, Befestigungsstelle oder vorgesehene Patientengruppe eines Sensors (100) oder Wandlers (200, 300) angeben, der bei der Überwachung physiologischer Parameter verwendet wird,
- eine Herzfrequenzschätzeinheit (132), die dazu konfiguriert ist, einen erwarteten Bereich der Herzfrequenz des Patienten anhand der erhaltenen Patientenkategorieinformationen und/oder den erhaltenen Sensor- oder Wandlerinformationen zu schätzen, und
- eine Einstelleinheit (133), die dazu konfiguriert ist, eine oder mehrere Einstellungen des Filters (11) basierend auf dem erwarteten Bereich der Herzfrequenz des Patienten zu erzeugen und den Filter (11) zum Filtern eines von der Vorrichtung (10, 20, 30) zur Überwachung physiologischer Parameter oder dem Sensor (100) gemessenen Sensorsignals basierend auf den erzeugten Einstellungen zu konfigurieren.

2. Einstellvorrichtung nach Anspruch 1,
wobei die Dateneingabe (131) dazu konfiguriert ist, die Patientenkategorieinformationen über eine Benutzereingabe oder aus einer Datenbank mit Patientendaten zu erhalten.

3. Einstellvorrichtung nach einem der vorstehenden Ansprüche,
wobei die Dateneingabe (131) dazu konfiguriert ist, die Sensor- oder Wandlerinformationen über eine Benutzereingabe oder durch Erkennen des Typs und/oder der Größe des Sensors oder Wandlers zu erhalten.

4. Einstellvorrichtung nach einem der vorstehenden Ansprüche,
wobei die Dateneingabe (131) dazu konfiguriert ist, zusätzlich den erwarteten Bereich der Herzfrequenz des Patienten über eine Benutzereingabe zu erhalten, und
wobei die Herzfrequenzschätzeinheit (132) dazu konfiguriert ist, den erwarteten Bereich der Herzfrequenz des Patienten aus einem Vergleich des geschätzten Bereichs und des erwarteten Bereichs, der über eine Benutzereingabe erhalten wurde, zu ermitteln.

5. Einstellvorrichtung nach einem der vorstehenden Ansprüche,
wobei die Einstelleinheit (133) dazu konfiguriert ist, basierend auf dem erwarteten Bereich der Herzfrequenz des Patienten einen digitalen Filter, einen linearen zeitinvarianten Filter oder einen adaptiven Filter auszuwählen oder einen Filter aus einer Gruppe vordefinierter Filter auszuwählen oder einen Satz von Einstellungen aus einer Gruppe von Sätzen von Einstellungen auszuwählen.

6. Einstellvorrichtung nach einem der vorstehenden Ansprüche,
wobei die Einstelleinheit (133) dazu konfiguriert ist, als eine oder mehrere Einstellungen eines oder mehrerer von Eckfrequenz eines Tiefpassfilters oder Hochpassfilters, Eckfrequenzen eines Bandpassfilters, Filterordnung, Filtertyp, maximaler Durchlassbandwelligkeit, Sperrbandwelligkeit, Fensterfunktion, Anpassungsalgorithmus, Ober- und/oder Untergrenzen für Filterkoeffizientenwerte, Obergrenzen für die 12-Filternorm, eine oder mehrere Einschränkungen, die gewisse Verstärkungen bei gewissen Frequenzen erzwingen, und durch den Filter zu minimierende und/oder zu maximierende Funktion zu erzeugen.

7. Einstellvorrichtung nach einem der vorstehenden Ansprüche,
wobei die Einstelleinheit (133) dazu konfiguriert ist, falls die Patientenkategorie Erwachsene sind, als Einstellung eine obere Eckfrequenz des Filters in einem Bereich zwischen 4 Hz und 5 Hz zu erzeugen, falls die Sensor- oder Wandlergröße einen Größenschwellenwert überschreitet, und als Einstellung eine obere Eckfrequenz des Filters in einem Bereich über 5 Hz zu erzeugen, falls die Sensor- oder Wandlergröße unterhalb eines Größenschwellenwerts liegt, insbesondere falls die Patientenkategorie Neugeborene oder Kleinkinder sind, als Einstellung eine obere Eckfrequenz des Filters in einem Bereich über 5 Hz zu erzeugen.

8. Einstellvorrichtung nach einem der vorstehenden Ansprüche,
wobei die Vorrichtung zur Überwachung physiologischer Parameter eine Blutdrucküberwachungsvorrichtung (30) ist, die zum Messen des Blutdrucks eines Patienten konfiguriert ist, der Wandler eine Manschette (300) ist, die Wandlerinformationen Manschettengrößeninformationen einschließen, die die Manschettengröße einer Manschette (300) angeben, und der Filter (11) zum Filtern eines von der Blutdrucküberwachungsvorrichtung (30) gemessenen Manschettendrucksignals konfiguriert ist; und
wobei die Dateneingabe (131) dazu konfiguriert ist, die Größe der Manschette durch Überwachen der Änderung des Manschettendrucks als Reaktion auf ein bestimmtes in die Manschette gepumptes Luftvolumen oder durch Identifizieren der Größe anhand einer in oder an der Manschette angeordneten Kennung zu erkennen.

9. Einstellvorrichtung nach einem der vorstehenden Ansprüche,
wobei die Einstelleinheit (133) dazu konfiguriert ist, die Patientenkategorieinformationen und/oder Sensor- oder Wandlerinformationen zu verwenden, um eine oder mehrere Einstellungen des Filters (11) zu erzeugen.

10. Computerimplementiertes Verfahren zum Konfigurieren eines Filters (11) in einer Vorrichtung (10, 20, 30) zur Überwachung physiologischer Parameter, die zum Messen eines physiologischen Parameters eines Patienten konfiguriert ist, wobei das Verfahren umfasst:
- Erhalten von Patientenkategorieinformationen, die die Patientenkategorie des Patienten angeben, und/oder von Sensor- oder Wandlerinformationen, die eines oder mehrere von Typ, Größe, Form, Befestigungsstelle oder vorgesehene Patientengruppe eines Sensors (100) oder Wandlers (200, 300) angeben, der bei der Überwachung physiologischer Parameter verwendet wird,
- Schätzen eines erwarteten Bereichs der Herzfrequenz des Patienten anhand der erhaltenen Patientenkategorieinformationen und/oder der erhaltenen Sensor- oder Wandlerinformationen,
- Erzeugen einer oder mehrerer Einstellungen des Filters (11) basierend auf dem erwarteten Bereich der Herzfrequenz des Patienten, und
- Konfigurieren des Filters (11) zum Filtern eines von der Vorrichtung (10, 20, 30) zur Überwachung physiologischer Parameter oder dem Sensor (100) gemessenen Sensorsignals basierend auf den erzeugten Einstellungen.

11. System (1, 2, 3) zur Überwachung physiologischer Parameter, umfassend:
- einen Sensor (100, 14, 33), der zum Erfassen eines Sensorsignals von einem Patienten konfiguriert ist;
- einen Filter (11), der zum Filtern des Sensorsignals konfiguriert ist;
- eine Vorrichtung (10, 20, 30) zur Überwachung physiologischer Parameter, die einen Prozessor (12, 34) umfasst, der zum Ermitteln eines physiologischen Parameters des Patienten basierend auf dem gefilterten Sensorsignal konfiguriert ist; und
- eine Einstellvorrichtung (13) nach einem der Ansprüche 1 bis 9, die zum Konfigurieren des Filters (11) konfiguriert ist.

12. System zur Überwachung physiologischer Parameter nach Anspruch 11, wobei
die Vorrichtung zur Überwachung physiologischer Parameter eine Blutdrucküberwachungsvorrichtung (30) ist, die zum Messen des Blutdrucks eines Patienten konfiguriert ist,
der Wandler eine Manschette (300) ist,
die Wandlerinformationen Manschettengrößeninformationen einschließen, die die Manschettengröße einer Manschette (10) angeben,
der Sensor (33) ein Drucksensor ist, der dazu konfiguriert ist, den Manschettendruck zu messen und ein Manschettendrucksignal zu erzeugen,
der Filter (11) dazu konfiguriert ist, ein von der Blutdrucküberwachungsvorrichtung (30) gemessenes Manschettendrucksignal zu filtern,
die Vorrichtung (30) zur Überwachung physiologischer Parameter weiter eine zum Aufblasen der Manschette (300) konfigurierte Druckerzeugungseinheit (31) umfasst, die dazu konfiguriert ist, an einem Körperteil des Patienten befestigt zu werden, sowie ein Ventil (32), das zum Entleeren der Manschette konfiguriert ist, und
der Prozessor (34) dazu konfiguriert ist, die Druckerzeugungseinheit (31) und das Ventil (32) zu steuern und den Blutdruck des Patienten basierend auf dem gefilterten Manschettendrucksignal zu ermitteln.

13. System zur Überwachung physiologischer Parameter nach Anspruch 11,
wobei der Sensor (100) ein Sensor für physiologische Parameter ist, der dazu konfiguriert ist, an einem Körperteil eines Patienten befestigt zu werden, insbesondere ein Herzfrequenzsensor, ein Atemfrequenzsensor oder ein Pulsoximetriesensor.

14. System zur Überwachung physiologischer Parameter nach Anspruch 11 oder 13,
wobei der Wandler (200, 300) eine nichtinvasive Blutdruckmanschette, ein Ultraschallwandler, ein photoplethysmographischer Wandler oder ein direkter Druckwandler ist.

15. Computerprogramm, das Programmcode umfasst, um einen Computer zu veranlassen, das Verfahren nach Anspruch 10 auszuführen, wenn das Computerprogramm auf dem Computer ausgeführt wird.

## Revendications

1. Dispositif de réglage (13) pour configurer un filtre (11) pour un dispositif de surveillance de paramètre physiologique (10, 20, 30) configuré pour mesurer un paramètre physiologique d'un patient, le dispositif de réglage comprenant :
- une entrée de données (131) configurée pour obtenir des informations de catégorie de patient indiquant la catégorie de patient du patient et/ou des informations de capteur ou de transducteur indiquant un ou plusieurs du type, de la taille, de la forme, de l'emplacement de fixation ou de la population de patients visée d'un capteur (100) ou d'un transducteur (200, 300) utilisé dans la surveillance de paramètre physiologique,
- une unité d'estimation de fréquence cardiaque (132) configurée pour estimer une plage attendue de la fréquence cardiaque du patient à partir des informations de catégorie de patient obtenues et/ou des informations de capteur ou de transducteur obtenues, et
- une unité de réglage (133) configurée pour générer un ou plusieurs réglages du filtre (11) sur la base de la plage attendue de la fréquence cardiaque du patient et pour configurer le filtre (11) pour filtrer un signal de capteur mesuré par le dispositif de surveillance de paramètre physiologique (10, 20, 30) ou le capteur (100) sur la base des réglages générés.

2. Dispositif de réglage selon la revendication 1,
dans lequel l'entrée de données (131) est configurée pour obtenir les informations de catégorie de patient au moyen d'une entrée utilisateur ou à partir d'une base de données contenant des données de patient.

3. Dispositif de réglage selon l'une quelconque des revendications précédentes,
dans lequel l'entrée de données (131) est configurée pour obtenir les informations de capteur ou de transducteur au moyen d'une entrée utilisateur ou en détectant le type et/ou la taille du capteur ou du transducteur.

4. Dispositif de réglage selon l'une quelconque des revendications précédentes,
dans lequel l'entrée de données (131) est configurée pour obtenir en outre la plage attendue de la fréquence cardiaque du patient au moyen d'une entrée utilisateur et
dans lequel l'unité d'estimation de fréquence cardiaque (132) est configurée pour déterminer la plage attendue de la fréquence cardiaque du patient à partir d'une comparaison de la plage estimée et de la plage attendue obtenue au moyen d'une entrée utilisateur.

5. Dispositif de réglage selon l'une quelconque des revendications précédentes,
dans lequel l'unité de réglage (133) est configurée pour sélectionner un filtre numérique, un filtre linéaire invariant dans le temps ou un filtre adaptatif ou pour sélectionner un filtre parmi un groupe de filtres prédéfinis ou pour sélectionner un ensemble de réglages parmi un groupe d'ensembles de paramètres sur la base de la plage attendue de la fréquence cardiaque du patient.

6. Dispositif de réglage selon l'une quelconque des revendications précédentes,
dans lequel l'unité de réglage (133) est configurée pour générer comme un ou plusieurs réglages un ou plusieurs de la fréquence de coin d'un filtre passe-bas ou d'un filtre passe-haut, de fréquences de coin d'un filtre passe-bande, d'un ordre de filtre, du type de filtre, de l'ondulation maximale de la bande passante, de l'ondulation de la bande d'arrêt, d'une fonction de fenêtre, d'un algorithme d'adaptation, de limites supérieures et/ou inférieures concernant des valeurs de coefficient de filtre, de limites supérieures pour la norme 12 de filtre, d'une ou plusieurs contraintes qui forcent certains gains à certaines fréquences et d'une fonction à minimiser et/ou à maximiser par le filtre.

7. Dispositif de réglage selon l'une quelconque des revendications précédentes,
dans lequel l'unité de réglage (133) est configurée, dans le cas dans lequel la catégorie de patient est adulte, pour générer comme réglage une fréquence de coin supérieur du filtre dans une plage entre 4 Hz et 5 Hz dans le cas dans lequel la taille de capteur ou de transducteur dépasse un seuil de taille et pour générer comme réglage une fréquence de coin supérieur du filtre dans une plage supérieure à 5 Hz dans le cas dans lequel la taille de capteur ou de transducteur est inférieure à un seuil de taille, en particulier pour générer, dans le cas dans lequel la catégorie de patient est néonatale ou jeune enfant, comme réglage une fréquence de coin supérieur du filtre dans une plage supérieure à 5 Hz.

8. Dispositif de réglage selon l'une quelconque des revendications précédentes,
dans lequel le dispositif de surveillance de paramètre physiologique est un dispositif de surveillance de pression artérielle (30) configuré pour mesurer la pression artérielle d'un patient, le transducteur est un brassard (300), les informations de transducteur incluent des informations de taille de brassard indiquant une taille de brassard d'un brassard (300), et le filtre (11) est configuré pour filtrer un signal de pression de brassard mesuré par le dispositif de surveillance de pression artérielle (30) ; et
dans lequel l'entrée de données (131) est configurée pour détecter la taille du brassard en surveillant le changement de pression de brassard en réponse à un volume particulier d'air pompé dans le brassard ou en identifiant la taille à partir d'un identifiant agencé dans le brassard ou au niveau de celui-ci.

9. Dispositif de réglage selon l'une quelconque des revendications précédentes,
dans lequel l'unité de réglage (133) est configurée pour utiliser les informations de catégorie de patient et/ou les informations de capteur ou de transducteur pour générer un ou plusieurs réglages du filtre (11).

10. Procédé mis en œuvre par ordinateur pour configurer un filtre (11) dans un dispositif de surveillance de paramètre physiologique (10, 20, 30) configuré pour mesurer un paramètre physiologique d'un patient, le procédé comprenant :
- l'obtention d'informations de catégorie de patient indiquant la catégorie de patient du patient et/ou d'informations de capteur ou de transducteur indiquant un ou plusieurs du type, de la taille, de la forme, de l'emplacement de fixation ou de la population de patients visée d'un capteur (100) ou d'un transducteur (200, 300) utilisé dans la surveillance de paramètre physiologique,
- l'estimation d'une plage attendue de la fréquence cardiaque du patient à partir des informations de catégorie de patient obtenues et/ou des informations de capteur ou de transducteur obtenues,
- la génération d'un ou plusieurs réglages du filtre (11) sur la base de la plage attendue de la fréquence cardiaque du patient, et
- la configuration du filtre (11) pour filtrer un signal de capteur mesuré par le dispositif de surveillance de paramètre physiologique (10, 20, 30) ou le capteur (100) sur la base des réglages générés.

11. Système de surveillance de paramètre physiologique (1, 2, 3) comprenant :
- un capteur (100, 14, 33) configuré pour acquérir un signal de capteur provenant d'un patient ;
- un filtre (11) configuré pour filtrer le signal de capteur ;
- un dispositif de surveillance de paramètre physiologique (10, 20, 30) comprenant un processeur (12, 34) configuré pour déterminer un paramètre physiologique du patient sur la base du signal de capteur filtré ; et
- un dispositif de réglage (13) selon l'une quelconque des revendications 1 à 9 configuré pour configurer le filtre (11).

12. Système de surveillance de paramètre physiologique selon la revendication 11, dans lequel
le dispositif de surveillance de paramètre physiologique est un dispositif de surveillance de pression artérielle (30) configuré pour mesurer la pression artérielle d'un patient,
le transducteur est un brassard (300),
les informations de transducteur incluent des informations de taille de brassard indiquant la taille de brassard d'un brassard (10),
le capteur (33) est un capteur de pression configuré pour mesurer la pression de brassard et générer un signal de pression de brassard,
le filtre (11) est configuré pour filtrer un signal de pression de brassard mesuré par le dispositif de surveillance de pression artérielle (30),
le dispositif de surveillance de paramètre physiologique (30) comprend en outre une unité de génération de pression (31) configurée pour gonfler le brassard (300) qui est configuré pour être attaché à une partie du corps d'un patient et une valve (32) configurée pour dégonfler le brassard, et
le processeur (34) est configuré pour commander l'unité de génération de pression (31) et la valve (32) et pour déterminer la pression artérielle du patient sur la base du signal de pression de brassard filtré.

13. Système de surveillance de paramètre physiologique selon la revendication 11,
dans lequel le capteur (100) est un capteur de paramètre physiologique qui est configuré pour être fixé à une partie du corps d'un patient, en particulier un capteur de fréquence cardiaque, un capteur de fréquence respiratoire ou un capteur d'oxymétrie de pouls.

14. Système de surveillance de paramètre physiologique selon la revendication 11 ou 13,
dans lequel le transducteur (200, 300) est un brassard de pression artérielle non invasif, un transducteur à ultrasons, un transducteur photopléthysmographique ou un transducteur de pression directe.

15. Programme informatique comprenant un code de programme pour amener un ordinateur à exécuter le procédé selon la revendication 10 lorsque ledit programme informatique est exécuté sur l'ordinateur.
